(19) Europäisches Patentamt
European Patent Office
Office européen des brevets

(11) Publication number: **0 176 792**
**A2**

# (12) EUROPEAN PATENT APPLICATION

(21) Application number: **85111124.5**

(22) Date of filing: **03.09.85**

(51) Int. Cl.$^4$: **G 01 N 33/52**

(30) Priority: **05.10.84 JP 208326/84**

(43) Date of publication of application:
**09.04.86 Bulletin 86/15**

(84) Designated Contracting States:
**DE FR GB SE**

(71) Applicant: **TERUMO KABUSHIKI KAISHA trading as TERUMO CORPORATION**
**44-1, 2-chome, Hatagaya Shibuya-Ku**
**Tokyo 151(JP)**

(72) Inventor: **Kaminagayoshi, Satoshi**
**Noguchi Mansion, 3-405 166-1, Nouchi Noguchi-cho**
**Kakogawa-shi Hyogo-ken(JP)**

(74) Representative: **Henkel, Feiler, Hänzel & Partner**
**Möhlstrasse 37**
**D-8000 München 80(DE)**

## (54) Test strip.

(57) A test strip having a detection-function portion supported on a flat substrate, wherein the detection-function portion comprises a flat detection portion capable of detecting a substance of interest in a given sample body fluid and a fluid absorbing portion disposed as adjoined to the lateral walls of the detection portion and adapted to absorb the sample body fluid adhering to the surface of the detection portion and the fluid absorbing portion possesses a greater fluid absorbing ratio than the detection portion.

EP 0 176 792 A2

# TEST STRIP

## BACKGROUND OF THE INVENTION

Field of the Invention:

This invention relates to a test strip to be used for clinical test of body fluids such as urine, blood, asctic fluid, and spinal fluid.

Description of the Prior Art:

The conventional test strip has comprised an oblong substrate provided at one end thereof with a holding portion and a detection portion formed on the surface of the substrate by impregnating the surface portion of the substrate with reagents for the reaction utilized for detection and drying the impregnated surface portion. Generally the clinical test by the use of a test strip is effected by immersing the test strip as held fast by the holding portion thereof in a container of a collected sample body fluid such as blood or urine. The test strip immersed in the sample fluid is then removed from the container and the detection portion of the test strip is examined for coloration due to the immersion to determine whether or not the sample fluid contains the particular substance sought after by the test. Among the clinical tests of the nature described above is counted a clinical test performed for detection of glucose in urine or blood, for example. These tests by the use of a test strip are highly significant in medical practices and clinical experiments.

The conventional test strip of the foregoing description is so constructed that when the test strip is pulled out of the sample body fluid, part of the body fluid adheres to the surface of the detection portion and tends to trickle down the substrate and smear the finger tips holding the holding portion because the holding portion is retained on the lower side during the observation of the detection portion for coloration and further because the detection portion has a limited capacity for absorbing the sample

fluid. Thus, the conventional test strip has the possibility of entailing this hygienic drawback.

In the test by the use of the conventional test strip, therefore, there has been followed the practice of causing the test strip, immediately after its removal from the sample body fluid, to be attentively rubbed against the edge of the fluid container in order that the excess fluid will be scraped and dropped back into the container by the edge. But the excess fluid cannot be scraped sufficiently. Further, the conventional test strip has suffered from the disadvantage that the color assumed by the detection portion is subject to variation by the kind of body fluid adhering thereto or by the particular item of test involved.

Recently, processed foodstuffs and beverages for refreshment richly contain ascorbic acid as vitamin C and ample ingestion of vitamin preparations is prevailing for the management of health. Thus, users of these articles contain vitamins richly in their blood and excrete excess vitamins in their urine. When such urine or blood is tested by the use of the test strip of the foregoing description, there is the disadvantage that because of high reducing property of ascorbic acid, the oxygen formed by peroxidase or a peroxidase-like substance is consumed in the oxidation-reduction reaction with ascorbic acid possibly to the extent of impeding the reaction with the oxidation indicator and obstructing ample coloration of the detection portion.

To overcome the disadvantage, I have already invented a method for preventing the detection of glucose and blood in a given sample urine from the influence of ascorbic acid by causing the detection portion of the test strip to be impregnated in advance with an oxidizing agent such as $NaIO_4$, $HIO_4$, or $CuSO_4$ in an amount necessary for the oxidation of ascorbic acid (U.S. Serial Number 633,959). This method, however, has the possibility that when the urine adheres amply to the detection portion and the urine happens to contains ascorbic acid in an amount more than can

be· oxidized with the aforementioned oxidizing agent, the portion of ascorbic acid which has escaped the oxidation prevents the detection part from being sufficiently colored.

An object of this invention, therefore, is to provide a hygienic test strip which does not suffer the sample body fluid such as blood or urine adhering to the detection portion and the substrate to trickle down the substrate and smear the holder's fingers.

Another object of this invention is to prevent the detection portion of the test strip from inducing an erroneous coloring reaction owing to excessive adhesion of sample body fluid.

## SUMMARY OF THE INVENTION

The objects described above are attained by a test strip having a detection-function portion supported on a flat substrate, wherein the detection-function portion comprises a flat detection portion capable of detecting a substance of interest in a given sample body fluid and a fluid absorbing portion disposed as adjoined to the lateral walls of the detection portion and adapted to absorb the body fluid adhering to the surface of the detection portion and the fluid absorbing portion possesses a greater fluid absorbing ratio than the detection portion.

This invention forms the detection portion of the test strip with a material permitting the body fluid to form a body fluid layer or body fluid pool on the surface thereof.

This invention has the fluid abosrbing portion formed in a substantially flat shape with one side thereof adjoining the substrate and the other side thereof incorporating the detection portion and gives the detection portion side of the fluid absorbing portion a greater surface area than the side adjoining the detection portion.

This invention has the detection portion and the fluid absorbing portion carried on the substrate and has the liquid absorbing portion adjoining the opposite sides of the

detection portion.

This invention also has the detection portion carried on one side of the substrate and has the fluid abosrbing portion carried on the other side of the substrate.

This invention further has the detection portion functioning as a part for detecting glucose in urine.

This invention has the detection portion functioning as a part for detecting occult blood in urine.

This has the detection portion functioning as a part for detecting objects in whole blood.

This invention also has the detection portion formed of a detection member and a water-impervious adhesive layer and has the detection member adhering to the fluid absorbing part through the medium of the adhesive layer.

BRIEF DESCRIPTION OF THE DRAWINGS

Fig. 1 is a cross sectional view illustrating a typical test strip as one preferred embodiment of the present invention,

Fig. 2 is a cross sectional view illustrating another typical test strip as another embodiment of this invention,

Fig. 3 is a cross sectional view of still another typical test strip as another embodiment of this invention,

Fig. 4 is a cross sectional view illustrating a further typical test strip as another embodiment of this invention,

Fig. 5 is a cross section taken through Fig. 5 along the line V-V,

Fig. 6 is a cross sectional view illustrating yet another typical test strip as another embodiment of this invention,

Fig. 7 is a perspective view illustrating the test of urine for glucose content by the use of a test strip of the present invention,

Fig. 8A is a plan view showing the test of urine

for glucose content by the use of a test strip,

Fig. 8B is a plan view of a color tone table to be compared with a test strip, and

Figs. 9 to 12 is graphs showing relations between concentrations of ascorbic acid and coloration.

DESCRIPTION OF PREFERRED EMBODIMENT

Now, preferred embodiments of the present invention will be described below with reference to the accompanying drawings.

Fig. 1 is a cross sectional view illustrating a typical test strip as one embodiment of the present invention. A test strip 10 has a flat oblong substrate 11 made of a resinous material such as polyester or polyamide and has one end portion of the substrate 11 serving as a holding portion 12. The substrate 11 has formed on the surface of the other end portion side thereof a detection-fuction portion 15 comprising a fluid absorbing portion 13 and a detection portion 14. The fluid absorbing portion 13 is formed of a fluid-absorbing synthetic resin and joined with adhesive agent or double-face adhesive tape. The detection portion 14 is formed in a buried state as juxtaposed to the fluid absorbing portion 13. The fluid absorbing portion 13 is possessed of a surface area greater than the surface of contact thereof with the detection portion 14. The detection portion 14 has been formed by impregnating a material such as filter paper or non-woven fabric with reagents corresponding to the particular item of test such as, for example, urobilinogen, protein, glucose, occult blood in urin, or objects in whole blood and then drying the impregnated material. It is applied fast on the fluid absorbing portion 13 such as with adhesive agent or a double face adhesive tape.

Fig. 2 is a cross sectional view illustrating a typical test strip as another embodiment of this invention. A test strip 20 possesses a flat oblong substrate 21 similar in construction to the substrate 11 of the test strip 10.

One end portion of the substrate 21 constitutes itself a pinch part 22. On the surface of the other end portion of the substrate 21 is formed a detection-function portion 25 similar to the detection-function portion 15 of the test strip 10. On the surface of a fluid absorbing portion 23, a water-impervious adhesive layer 26 is formed and a detecting member 27 similar in function to the detector portion 14 of the embodiment is buried as adjoined to the fluid absorbing portion 23 through the medium of the adhesive layer 26 to give rise to a detection portion 24. The fluid absorbing part 23 has a surface area larger than the surface of contact thereof with the adhesive layer 26.

Fig. 3 is a cross sectional view illustrating a typical test strip as another embodiment of the present invention. A test strip 30 possesses a flat oblong substrate 31 similar in construction to the substrate 11 of the test strip 10. One end portion of the substrate 31 constitutes itself a holding portion 32. On the surface of the other end portion side of the substrate 31, a detection-function part 35 similar to the detection-function portion 15 of the embodiment is disposed. A fluid absorbing portion 33 adjoins the opposite sides of a detection portion 34 and joined to the substrate 31 such as with adhesive agent or a double face adhesive tape in such a manner that the surface therof falls flush with the surface of the detection portion 34.

Figs. 4 and 5 are a cross sectional view and a front view illustrating a typical test strip as another embodiment of this invention. A test strip 40 is possesses of a flat oblong substate 41 similar in construction to the substrate 11 of the test strip 10. One end portion of the substrate 41 constitutes itself a holing portion 42. On the surface of the other end portion similar to the detection-function portion 15 of the embodiment is disposed. A detection portion 44 is formed on the substrate 41 and a fluid absorbing portion 43 is formed on the other side of

the substrate 41 so that the two portions nip the substrate therebetween. These two portions are both fastened to the substrate 41 with adhesive agent or a double face adhesive tape. The fluid absorbing portion 43 has a surface area greater than the surface area of the detection portion 44.

Fig. 6 is a cross sectional view illustrating a typical test strip as still another embodiment of this invention. A test strip 50 is possessed of a flat oblong substrate 51 similar in construction to the substrate 11 of the test portion 10. One end portion of this substrate 51 constitutes a holding portion 52. On the surface of the substrate 51 is formed a detection-function portion 55. This detection-function portion 55 comprises detection portions 54, 56, 57, and 58 formed by impregnation with reagents corresponding to varied items of test. On both sides of the detection portion 54 intended for the detection of glucose are juxtapositionally formed fluid absorbing portions 53 and 53. On the outer sides of these fluid absorbing portions 53 and 53 are disposed a detection portion 56 for the detection of protein, a detection portion 57 for the detection of occult blood, and a detection portion 58 for the detection of urobilinogen, for example. All these detection portions are invariably fastened to the substrate 51 such as with adhesive agent or a double face adhesive tape.

It should be noted that in Figs. 1 through 6, the test strips are invariably depicted in exaggerated thickness. The substrates are made of polystyrene, polyethylene terephthalate, polybutylene terephthalate, polyamide, polyethylene, polypropylene, or vinyl chloride resin, for example. The detection portions are formed by impregnating such substratal material as filter paper or non-woven fabric with the solution of a reagent used for the test and subsequently drying the impregnated substratal material. The fluid absorbing portions are formed for the purpose of removing by absorption the excess body fluid absorbed in the

detection portions. They are, therefore, required to be formed of a material which has a higher fluid absorbing ratio that the material of the detection portions and has an ability to absorb the body fluid trickling down from the detection portions or remain undissolved by the body fluid during the course of test such as, for example, for a period of 1 to 10 minutes. Typical examples of the material answering the description are polyvinyl alcohol, partially saponified polyvinyl acetate, polyacrylamide, sodium polyacrylate, and carboxymethyl cellulose.

Now, the method for the use of the test strips shown in the various embodiments mentioned above will be described below specifically with respect to the test strip of Fig. 3 taken as an example by consulting Figs. 7, 8A and 8B.

First the test strip 30 as held fast by the holding portion 32 is immersed in urine 61 sampled and placed in a container 60. During the immersion, the test strip is held in such a manner that the holding portion 32 will remain on the upper side and the test strip 30 is pushed in the urine 61 until the detection portion 34 thereof is completely covered with the urine 61.

The test strip 30 so immersed in the urine 61 is withdrawn from the urine, then held in a state having the holding portion 32 on the lower side as illustrated in Fig. 8A, and subjected to observation as to the outcome of coloration reaction. This observation is effected by comparing the detection portion of this test strip 30 with the color tone table 62 shown in Fig. 8B. Through this observation of the outcome of the coloration reaction manifested on the detection portion 34, the concentration in the urine 61 of the substance of interest being sought by the particular item of test is determined. The test strips 10, 20, 40 and 50 are used in much the same was as the test strip 30 described above to permit determination of concentrations of substances of interest corresponding to

particular items of test.

When the test strip 10, 20, 30, 40 and 50 of the various embodiments described above are immersed in the urine 61 and then removed therefrom, the excess portions of urine 61 adhering to their substrates 11, 21, 31, 41 and 51 and their detection portions 14, 24, 34, 44 and 54 are eventually absorbed by their fluid absorbing portions 13, 23, 33, 43 and 53. Subsequently, the test strips 10, 20, 30, 40 and 50 are held in place with their holding portions 12, 22, 32, 42 and 52 kept on the lower side as illustrated in Fig. 8A to compare the detection portions 14, 24, 34, 44, and 54 with the color tone table 62. During the observation of these detection portions 14, 24, 34, 44, and 54, the excess portions of urine 61 trickling down the substrates 11, 21, 31, 41, and 51 are thoroughly absorbed by the fluid absorbing portions 13, 23, 33, 43, and 53 of a high fluid absorbing ratio and no portion of urine 61 is suffered to reach the finger tips holding the holding portions 12, 22, 32, 42, and 52. Thus, the test strips 10, 20, 30, 40, and 50 warrant hygienic handling.

In the test strips 10, 20, 30, 40, and 50 of the various embodiments described above, the detection portions 14, 24, 34, 44, and 54 are disposed as adjoined to the fluid absorbing portions 13, 23, 33, 43, and 53. When the fluid absorbing portions 13, 23, 33, 43, and 53 are given large surface areas as compared with the detection portions 14, 24, 34, 44, and 54, the body fluid such as urine or blood which happens to adhere excessively to the surface portions of the detection portions 14, 24, 34, 44, and 54 is safely and easily absorbed by the fluid absorbing portions 13, 23, 33, 43, and 53. If this body fluid happens to have a color as in the case of blood, it never happens that the body fluid adheres in the form of film to the surfaces of the detection portions 14, 24, 34, 44, and 54, rendering easy the observation of the outcome of coloration reaction as compared with the conventional test strip. Particularly in

the case of the test strip 30, 50 of the third and fifth embodiments described above, since the detection portions 34, 54 have their surface flush with the surfaces of the fluid absorbing portions 33, 53, the body fluid which happens to adhere to the surfaces of the detection portions 34, 54 is safely absorbed out of the boundaries of the detection portions 34, 54 into the fluid absorbing portions 33, 53.

In the conventional test strip, it has often happened that, depending of the item of test, the relevant body fluid adheres excessively to the detection portion and prevents the ensuing coloration reaction from giving an exact result. For example, the test of urine for glucose or occult blood has entailed the disadvantage mentioned above. The test for such substance, therefore, has been carried out as follows.

The detection of glucose in urine is based on the following principle.

$$\text{Glucose (in urine)} \xrightarrow{\text{"Glucose oxidase"}} H_2O_2 \xrightarrow{\text{"Peroxidase"}} O_2 \rightarrow \text{Coloration reaction by indicator}$$

Thus, the detection of glucose in urine is carried out by using a test strip having the detection portion thereof impregnated with glucose oxidase and peroxidase. If a given sample urine happens to contain glucose, this glucose is converted into $H_2O_2$ by the action of the enzyme "glucose oxidase" incorporated in the detecting portion. Further, by the action of the other enzyme "peroxidase," the $H_2O_2$ is converted into $O_2$. As the result, the oxygen $O_2$ induces the indicator to produce a color.

Then, the detection of occult blood in urine is based on the following principle.

$$\text{Peroxide} \xrightarrow{\text{Hemoglobin (Peroxidase)}} O_2 \longrightarrow \text{Coloration reaction by indicator}$$

In the test strip 10, 20, 30, 40, and 50 offered

by the various embodiments described above, since the detection portions 14, 24, 34, 44, and 54 are disposed as adjoined to the fluid absorbing portions 13, 23, 33, 43, and 53, the detection portions 14, 24, 34, 44, and 54 are supplied with the body fluid in a proper amount necessary for the coloration reaction.

The detection of blood in urine is carried out by using a test strip having the detection portion thereof impregnated with peroxide. If a given sample urine happens to contain blood, the peroxide incorporated in the detection portion is induced to produce $O_2$ (because the hemoglobin in blood has a peroxidase-like activity). This oxygen $O_2$ induces the indicator to produce a color.

As pointed out previously, I have proposed use of an oxidizing agent such as $NaIO_4$, $HIO_4$, or $CuSO_4$ for the purpose of eliminating the influence of ascorbic acid present in the body fluid such as urine.

In the test strips of th various embodiments described above, when the detection portions 14, 24, 34, 44 and 54 prepared specifically for the detection of glucose or occult blood are formed on the surfaces of the fluid absorbing portions 13, 23, 33, 43, and 53 respectively, the detection portions 14, 24, 34, 44, and 54 are supplied with the urine in a proper amount for the oxidizing agent incorporated therein. The excessive portions of body fluid are eventually absorbed by the fluid abosrbing portions 13, 23, 33, 43, and 53. As the result, the detection can be carried out precisely with thorough elimination of the influence of ascorbic acid. Further oxygen which is necessary for formation of $H_2O_2$ can be entrapped from air.

Further, in the test strip of the embodiments described above, the surfaces of the detection portions 14, 24, 34, 44, and 54 are supplied with the body fluid such as urine or blood in a uniform amount. As the result, the reaction proceeds uniformly in the detection portions. Thus, the test strips permit desired tests to be carried out

with added accuracy.

In the test strip 20 of the second embodiment, since the water-impervious adhesive layer is interposed between the detection portion 24 and the fluid absorbing portion 23, the body fluid absorbed excessively in the fluid absorbing portion 23 is never allowed to pass into the detection portion 24. Thus, this test strip 20 permits a given test to be carried out accurately.

Test strips incorporating fluid absorbing portions as contemplated in the embodiments of this invention were immersed in a solution of glucose and ascorbic acid and then examined for the outcome of coloration reaction. Comparative test strips of the conventional principle were similarly treated and examined. The results were compared.

The test strips used herein were specifically prepared for the detection of glucose in urine.

The detection portions in these test strips were produced by impregnating filter papers as substrates with the solution A indicated below, drying the impregnated substrates, then impregnating them with the solution B indicated below, and drying.

| | | |
|---|---|---|
| Solution A: | Citrate buffer solution | 100 ml |
| | Glucose oxidase | 300 mg |
| | Peroxidase | 50 mg |
| | Oxidizing agent ($NaIO_4$) | 100 mg |
| | Sodium arginate | 500 mg |
| Solution B: | Ortho-tolidine | 2.5 g |
| | /Acetone | 100 ml |

A test strip, X, of the construction shown in Fig. 3 was produced by using the detector portion described above. In this case, the substrate 31 was made of polystyrene terephthalate and the fluid absorbing portion 33 was made of polyvinyl alcohol of a water absorbing ratio 1,230% (product of Kanebo Synthetic Chemical, marketed under trademark designation of Bellclin) and applied fast to the substrate with a double face adhesive tape.

Another test strip, Y, similar to the conventional test strip was produced by directly applying the aforementioned detection portion on the surface of a substrate similar in construction to the aforementioned substrate 31.

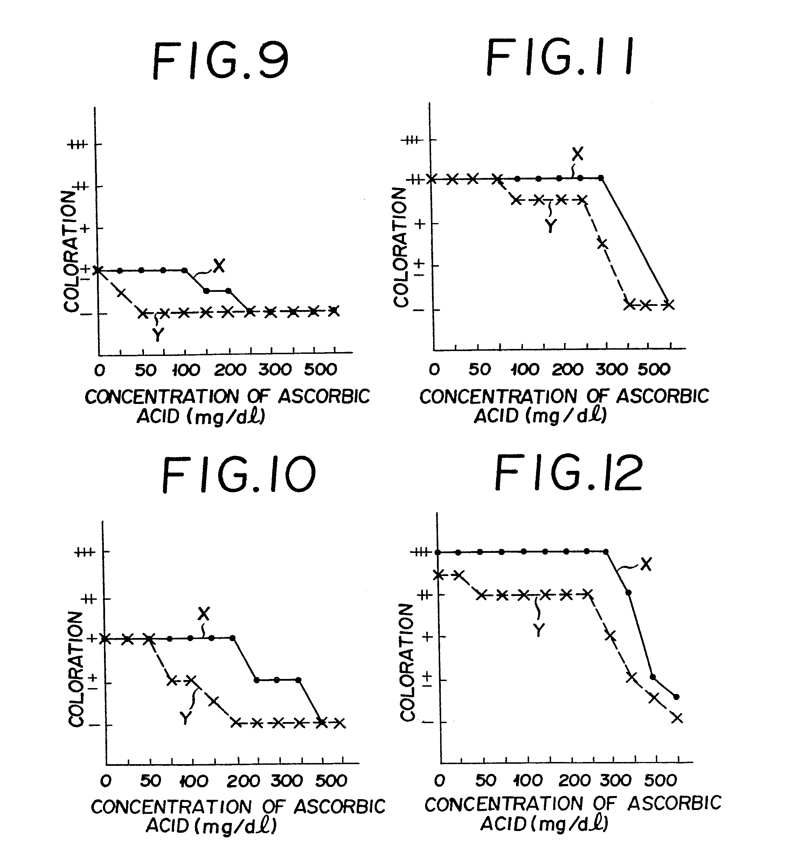

These test strips, X and Y, were immersed in a solution containing 50, 150, 500 and 2,000 mg of glucose and 0, 50, 100, 200, 250, 400, 500 and 750 mg of ascorbic acid respectively per dl by way of trial detection of glucose. The results are shown in Figs. 9 to 12. Fig. 9 shows an example of 50 mg/dl of glucose (shown by $\pm$), Fig. 10 shows an example of 150 mg/dl of glucose (shown by +), Fig. 11 shows an example of 500 mg/dl of glucose (shown by ++) and Fig. 12 shows and example of 2,000 mg/dl of glucose (shown by +++).

From the test results, it is noted that the test strip X which accords with the present invention is capable of supplying the detection portion with urine in a proper amount necessary for the reaction intended. It is further noted that the test strip Y supplies the detection portion with urine in an amount more than necessary for the reaction, rendering accurate test difficult.

Further in the test strip X, no overflow of body fluid was observed even when the pinch portion 32 was held on the lower side. In the test strip Y, the body fluid trickled down the substrate and smeared the holder's hand when the holding portion was held on the lower side.

As described above, the present invention concerns a test strip having a detection-function portion carried on a substrate, where in the detection-function portion comprises a detection portion and a fluid absorbing portion, the fluid absorbing portion is disposed as adjoined to the lateral walls of the detection portion, and the fluid absorbing portion has a higher fluid absorbing ratio than the detection portion. Thus, the test strip of this invention has no possibility of the body fluid such as blood or urine under test adhering excessively to the detection

portion or to the substrate and the body fluid trickling down the substrate and smearing the finger tips holding the holding portion thereof. This invention, accordingly, provides hygienic test strips.

Since this invention gives the fluid absorbing portion a surface area larger than the surface of contact between the fluid absorbing portion and the detection portion, the fluid absorbing portion is allowed to absorb the body fluid in a proper amount more easily than elsewhere.

Since this invention has the fluid absorbing portions disposed on both sides of the detection portion and has all these portions formed in one and the same height, the detection portion is enabled to have the body fluid adhere thereto in a proper amount necessary for the reaction the test strip is expected to undergo.

Further since this invention has the detection portion specifically prepared for the detection of glucose in urine, it permits the test of urine to be carried out precisely and hygienically.

Since this invention has the detection portion specifically prepared for the detection of occult blood in urine, it permits the test of urine to be carried out precisely and hygienically.

Since this invention has the detection portion specifically prepared for the detection of objects in whole blood, it permits the test of blood to be carried out precisely and hygienically.

Since this invention has the water-impervious adhesive layer interposed between the detection portion and the fluid absorbing portion, it permits the test to be carried out accurately without the possibility of the proper amount of the body fluid absorbed in the fluid absorbing portion passing into the detection portion.

This invention permits the detection portion to be formed of filter paper and the fluid absorbing portion to be

formed of a water absorbing polymer having a higher fluid absorbing ratio than the filter paper. In this arrangement, since the filter paper itself has a relatively low fluid absorbing ratio despite its high fluid absorbing speed, the absorbed body fluid is liable to flow down. However, since the detection portion is adjoined to the lateral walls of the fluid absorbing portion made of an absorbent polymer of high fluid absorbing ratio, the body fluid is absorbed by the fluid absorbing portion and is not suffered to trickle down. The adverse phenomena such as overflow of excessive body fluid and influence of ascorbic acid in urine are substantially eliminated by this invention.

WHAT IS CLAIMED IS:

1. A test strip having a detection-function portion supported on a flat substrate, wherein said detection-function portion comprises a flat detection portion capable of detecting a substance of interest in a given sample body fluid and a fluid absorbing portion disposed as adjoined to the lateral walls of said detection portion and adapted to absorb said sample body fluid adhering to the surface of said detection portion and said fluid absorbing portion possesses a greater fluid absorbing ratio than said detection portion.

2. A test strip according to Claim 1, wherein said fluid absorbing portion is in a substantially flat shape and has one side thereof adjoined to said substrate and the other side thereof superposed by said detection portion and said fluid absorbing portion has a larger surface area on the detection portion side thereof than the surface of contact with said detection portion.

3. A test strip according to Claim 1, wherein said detection portion and said fluid absorbing portion are supported on said substrate and said fluid absorbing portion is adjoined to the opposite sides of said detection portion.

4. A test strip according to Claim 1, wherein said detection portion is supported on one side of said substrate and said fluid absorbing portion is supported on the other side of said substrate.

5. A test strip according to Claim 1, wherein said detection portion is specifically prepared for the detection of glucose in urine.

6. A test strip according to Cliam 1, wherein said detection portion is specifically prepared for the detection of occult blood in urine.

7. A test strip according to Claim 1, wherein said detection portion is specifically prepared for the detection of objects in whole blood.

8. A test strip according to Claim 2, wherein said

detection portion comprises a detecting member and a water-impervious adhesive layer and said detecting member is applied fast to said fluid absorbing portion through the medium of said adhesive layer.

9. A test strip according to Claim 1, wherein said detection portion is formed of filter paper and said fluid absorbing portion is made of an absorbent polymer having a higher fluid absorbing ratio than said filter paper.

0176792

FIG.1

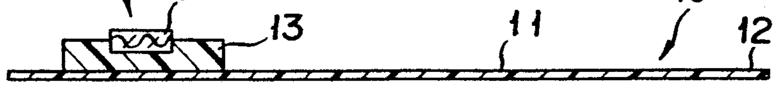


FIG.2

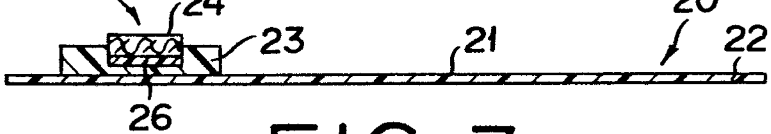


FIG.3

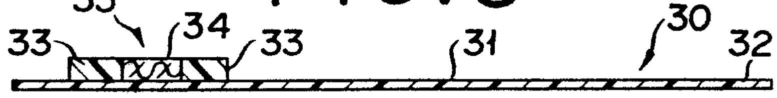


FIG.4

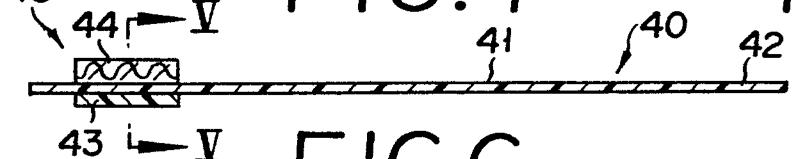


FIG5

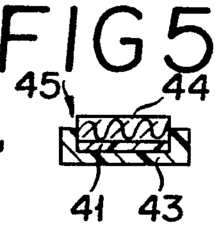


FIG.6

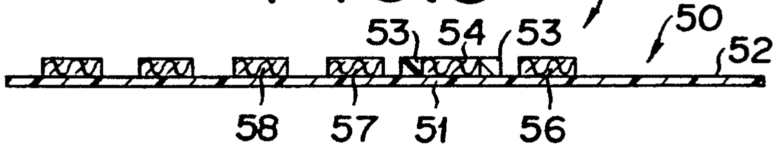


FIG.7

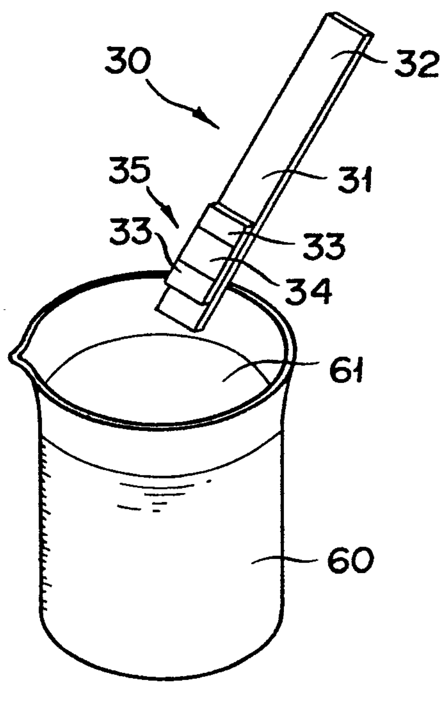


FIG.8A

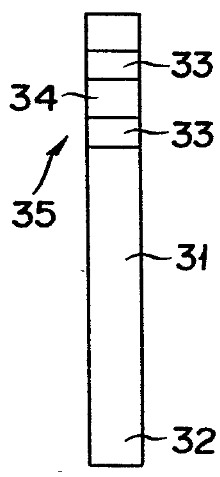


FIG.8B

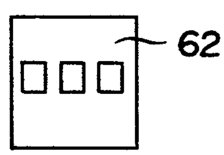

FIG.9

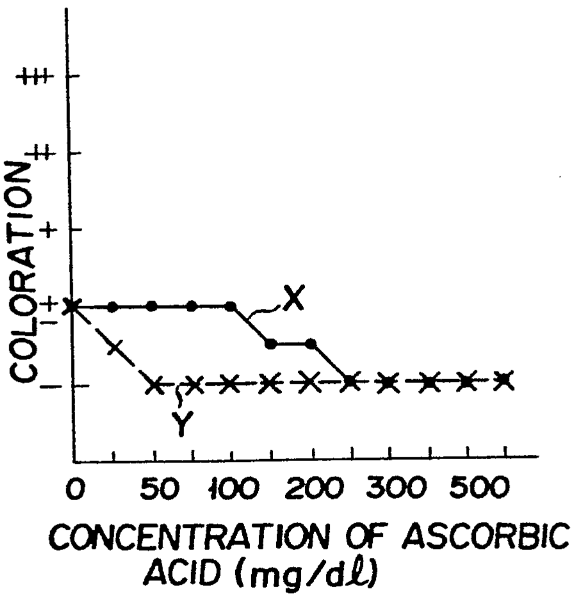


FIG.11

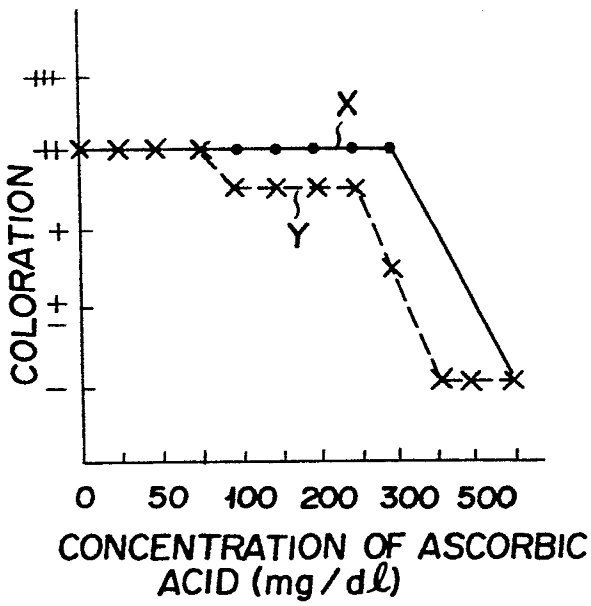


FIG.10

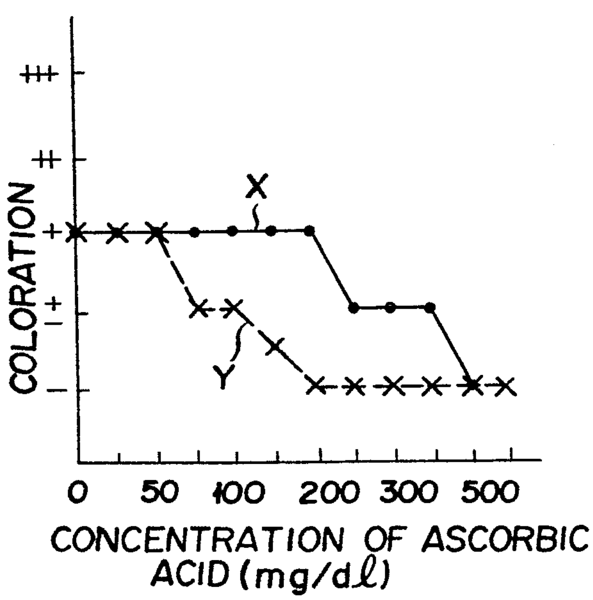


FIG.12